# EUROPEAN PATENT SPECIFICATION

(11) **EP 2 496 215 B1**
(45) Date of publication and mention of the grant of the patent: **14.01.2015**
(21) Application number: 10785173.5
(22) Date of filing: 01.11.2010
(51) Int. Cl.: A61K 9/08, A61P 17/10, A61P 31/04, A61K 47/08, A61K 47/12, A61K 47/14, A61K 47/10, A61K 31/343

(54) **ANTIMICROBIAL AND ANTI-ACNE FORMULATIONS**
ANTIMIKROBIELLE UND ANTIAKNE FORMULIERUNGEN
FORMULATIONS ANTIMICROBIELLES ET ANTI-ACNE

(30) Priority: 03.11.2009 GB 0919210
(43) Date of publication of application: 12.09.2012
(73) Proprietor: Venn Life Sciences Holdings plc, London EC3V 9HD (GB)
(72) Inventor: DAVIS, Adrian, Wetherby West Yorkshire LS22 7TW (GB); DONOGHUE, Gavin, Wetherby West Yorkshire LS22 7TW (GB); EADY, Elizabeth, Wetherby West Yorkshire LS22 7TW (GB)
(74) Representative: Brewster, Andrea Ruth
(86) International application number: PCT/GB2010/051824
(87) International publication number: WO 2011/055139

(56) References cited:
- EP-A1- 1 374 903
- GB-A- 2 456 376
- GB-A- 2 465 633
- US-A1- 2008 233 145
- DATABASE WPI Week 199723 Thomson Scientific, London, GB; AN 1997-255390 XP002663365, & JP 9 087106 A (CHUBU DENRYOKU KK) 31 March 1997 (1997-03-31) & JP 9 087106 A (CHUBU ELECTRIC POWER; TOKAI CONCRETE KOGYO KK) 31 March 1997 (1997-03-31)
- DATABASE WPI Week 199344 Thomson Scientific, London, GB; AN 1993-347478 XP002663367, & JP 5 246876 A (NIPPON PAINT CO LTD) 24 September 1993 (1993-09-24) & JP 5 246876 A (NIPPON PAINT CO LTD) 24 September 1993 (1993-09-24)

## Description

### Field of the invention

This invention relates to antimicrobial and anti-acne formulations.

### Background to the invention

Usnic acid is a naturally occurring dibenzofuran derivative which can be isolated from several species of lichen, in particular *Usnea.* It is also known as usneine, usninic acid and 2,6-diacetyl-7,9-dihydroxy-8,9b-dimethyl-1,3(2H,9bh)-dibenzofurandione.

Usnic acid is known to possess antimicrobial activity, including against Gram-positive bacteria and certain fungi. It is also believed to possess anti-inflammatory and analgesic activity. The acid and its salts (in particular sodium usnate) have been used in pharmaceuticals, cosmetics and perfumes, both as active ingredients and as preservatives.

Although usnates have been found to be potent against propionibacteria such as *P*. *acnes,* which are implicated in inflammatory acne, their size and relatively high melting points tend to reduce their skin penetrating ability, and in turn their therapeutic efficacy when applied topically *in vivo.* Thus, the formulation of an usnate for topical antimicrobial use, in particular to treat acne, presents challenges, not least the need to target the active to the infundibula of pilosebaceous follicles, where the propionibacteria reside, and to assist its skin penetration. Ideally the active should be formulated to penetrate through all the layers of the stratum corneum.

A further difficulty which accompanies the formulation of usnates is their low solubility in most common solvents. Their poor skin penetrating abilities mean that they need to be formulated at relatively high concentrations for topical delivery, yet there are few materials able to solubilise usnates at the concentrations required.

US2008/0233145 A1 discloses pharmaceutical compositions of *Usnea barbata* and the use thereof for the treatment of skin diseases. EP1374903 A1 discloses pharmaceutical compositions for the prevention of the development and progression of mycotic skin surface diseases. The compositions disclosed in EP1374903 comprise deo-usnate.

It is an aim of the present invention to provide a novel usnic acid- or usnate-containing formulation, which is suitable for topical application to the skin as an antimicrobial agent and in particular to treat acne.

### Statements of the invention

According to a first aspect of the present invention there is provided an antimicrobial and/or anti-acne formulation containing either usnic acid or an usnate, together with: (a) a primary solvent or solvent system in which the usnic acid or usnate is at least partially dissolved; (b) a glycol; and (c) a hydrophobic component (c) selected from fatty acids, fatty alcohols, fatty acid esters, and mixtures thereof, wherein the formulation is in the form of a liquid and contains 1% w/w or less of water.

The formulation may also contain (d) an alkylene glycol derivative, in particular Transcutol™.

This combination of excipients has been specifically designed to enhance the penetration of the usnic acid or usnate active into the skin, as well as to solubilise the active at therapeutically effective concentrations. The combination is also designed to be miscible with sebum. The active is at least partially solubilised in the primary solvent or solvent system (a). The components (b) and (c) are used respectively as partition coefficient and diffusion coefficient co-enhancers: they can help to optimise the partitioning of the active between the formulation and the stratum corneum, and its diffusion within the stratum corneum.

For use as a topical antimicrobial agent, in particular to treat acne, an active needs to be targeted to the infundibula of pilosebaceous follicles. It can reach these target sites by two routes: radial diffusion via the stratum corneum and direct delivery down the follicular ducts. The interplay between the two routes during drug delivery depends upon the physicochemistry of the active in any given case, but for usnic acid and usnate formulations, penetration via the stratum corneum is likely to be important. It is also now believed that miscibility of the formulation with sebum can help to optimise delivery overall.

On topical application to the skin, the levels of components (b) and (c) present in the stratum corneum can be higher than if the two components were delivered individually in the same amounts. Thus, there appears to be a synergistic interaction between the two components, which can lead to enhanced skin penetration for the formulation of the invention.

If present, the alkylene glycol derivative (d) can act as an additional partition coefficient co-enhancer, though typically weaker than the glycol (b). It can also increase the miscibility of the glycol and the hydrophobic component (c), making it possible to include higher concentrations of (c) if necessary. Moreover, it can be used to help dissolve the usnic acid or usnate: in other words, it can supplement the primary solvent or solvent system (a). Thus the concentration of the component (d) may depend on the nature and concentration of component (a).

The usnic acid or usnate active is suitably present in the mixture of components (a) to (d) at a concentration which is at or close to (ie at least 80 or 85 or 90 or 95% of, for instance from 80 to 95% of or from 80 to 90% of) saturation. This can increase the tendency of the active to partition out of the formulation and into the skin, and in general a saturated or supersaturated solution can help to optimise delivery of the dissolved active to the skin. In cases however the active may be present in the mixture of (a) to (d) at 50% or greater of its saturated concentration. Since (super)saturated solutions can be unstable over longer storage periods, in particular if exposed to changes in temperature, it may be advantageous to achieve the desired degree of saturation by formulating the active at a slightly lower concentration initially to allow for loss of volatile solvents during storage.

The concentration of the active which constitutes saturation will depend on the natures and concentrations of the other components of the formulation. It can be determined in conventional manner for any chosen combination of components. For example, the active can be added to the chosen combination of components to a level which is clearly in excess of its solubility limit (as will be evidenced by a degree of active precipitation). The excess, undissolved active can then be removed from the formulation by filtration, following which the formulation can be assayed to determine the concentration of active remaining. This concentration will represent the level at which the formulation is saturated with the active.

The hydrophobic component (c) is also suitably present at a concentration which is at or at least close to (ie at least 80 or 85% of, or at least 90 or 95 or 98% of) saturation, this being based on its concentration in the overall formulation (ie typically in the components (a), (b) and if present (d)). This can increase the tendency of the hydrophobic component to partition out of the formulation, with the usnic acid or usnate, and into the skin. Again, it may be desirable to achieve the desired degree of saturation by formulating the component (c) at a slightly lower concentration initially to allow for changes during storage. The concentration of component (c) which constitutes saturation can be determined in the way described above, and again will depend on the natures and concentrations of the other components of the formulation.

Thus, one method for designing a formulation according to the invention is to start with a desired usnic acid or usnate concentration, and then to identify a combination of the components (a) to (d) in which that concentration of usnic acid or usnate represents a saturated or nearly saturated system. The natures and concentrations of components (a) and if appropriate (d) may be adjusted in order to modify the solubility of the active and/or the component (c).

It is believed, although we do not wish to be bound by this theory, that the components (a) to (d), in particular when present in the preferred concentration ranges referred to below, can act together synergistically to assist penetration of the usnic acid or usnate into the skin. Moreover their relative proportions can be chosen to allow a relatively high concentration of the active to be carried in the formulation.

In a formulation according to the invention, the primary solvent or solvent system (a) may be any fluid or mixture of fluids in which the usnic acid or usnate salt can be at least partially dissolved. The choice of solvent will depend on the intended use of the formulation and its desired physical form, as well as on the natures and concentrations of the other components present and the required concentration of usnic acid or usnate in the overall formulation. It may be volatile or non-volatile, aqueous or non-aqueous.

It may itself comprise a mixture of two or more solvents. As described above, it may contain an alkylene glycol derivative such as Transcutol™.

Usnic acid and usnates can be poorly soluble in many commonly available solvents. The solvents which are of use in a formulation according to the invention are typically those which are able to dissolve 1% w/w or greater of the relevant active, or those which are able to dissolve 2 or 3 or 4 or 5% w/w or greater. Examples of suitable primary solvents include dimethyl isosorbide (DMI); ketones such as acetone, methyl ethyl ketone, diethyl ketone, butanone and 3-pentanone; alcohols such as ethanol, phenoxyethanol, isopropyl alcohol, 1-methoxy-2-propanol, benzyl alcohol and tetrahydrofurfuryl alcohol; polyalkylene glycols, in particular polyethylene glycols such as PEG 200, PEG 300 and PEG 400; other glycols such as hexaethylene glycol; polyalkoxylated (in particular polyethoxylated) esters; propylene phenoxytol; dimethyl formamide (DMF); and dimethyl sulphoxide (DMSO).

Examples of suitable polyalkoxylated esters include polyalkylene glycol stearates and hydroxystearates such as Solutol® HS 15 (PEG-15-hydroxystearate). They also include polyalkoxylated glyceryl esters (also known as a polyalkoxylated glycerides), such Glycerox™ esters (for example Glycerox™ 767) and Labrasol™. In an embodiment, the polyalkoxylated ester is Labrasol™.

A primary solvent system (a) may include a mixture of two or more of the above solvents. For example, a mixture of DMI and ethanol (such as a 1:1 mixture) may be used as the solvent system.

In an embodiment of the invention, the component (a) may be selected from DMI, benzyl alcohol, butanone, 3-pentanone, acetone, tetrahydrofurfuryl alcohol (THFA), phenoxyethanol and mixtures thereof. It may be selected from DMI, benzyl alcohol, butanone and mixtures thereof, all of which are able to dissolve copper usnate for example to greater than 2.5% w/w. In some cases, it may be preferred to avoid volatile solvents such as acetone.

In an embodiment, the component (a) is selected from DMI, butanone, phenoxyethanol, THFA and mixtures thereof. In an embodiment it is selected from DMI, butanone and mixtures thereof. In an embodiment it is DMI, in particular where the active is copper usnate since copper usnate appears to be more soluble in DMI than in many other common solvents. DMI may also enhance partition of the active out of the component (a).

It has surprisingly been found that when a primary solvent such as those listed above is added to the components (b) and (c), and in particular to a mixture of components (b) to (d), the result can be a higher than expected increase in the ability of the system as a whole to solubilise an usnic acid or usnate active. In other words, the addition of a relatively low concentration of a solvent (a), to a mixture of (b) and (c) or of (b), (c) and (d), can yield a greater than expected increase in the amount of the active which the formulation can carry. This is advantageous in the case of usnic acid and usnates, which as described above often need to be applied in relatively high doses in topical formulations.

The glycol (b) suitably has a molecular weight of 200 or less, or of 150 or 100 or less. It may for example be selected from ethylene glycols and propylene glycols, such as mono-ethylene glycol, diethylene glycol, triethylene glycol, mono-propylene glycol, 1,3-propylene glycol, dipropylene glycol, tripropylene glycol and mixtures thereof. Other suitable glycols include 1,2-propanediol; 1,4-butanediol; 2,3-butanediol; 1,3-butanediol; 1,5-pentanediol; 1,2-pentanediol; butylene glycol; pentylene glycol; 1,2-hexylene glycol; neopentyl glycol; and mixtures thereof. The component (b) may be a mono- or di-alkylene glycol, for example a mono- or di-(C1-C6 or C2-C4 alkylene) glycol, in particular a mono- or di-ethylene or propylene glycol.

In an embodiment, the glycol (b) is selected from ethylene, propylene and butylene glycols and mixtures thereof; or from propylene and butylene glycols and mixtures thereof. In an embodiment, the glycol (b) is selected from mono-propylene glycol, dipropylene glycol, 1,3-butanediol and mixtures thereof. In an embodiment it is a propylene glycol, for example selected from mono-propylene glycol, dipropylene glycol and mixtures thereof. In an embodiment, it is mono-propylene glycol. In an embodiment, it is 1,3-butanediol (butylene glycol).

The hydrophobic component (c) is selected from fatty acids, fatty alcohols, fatty acid esters, and mixtures thereof. In an embodiment, it is a fatty alcohol or fatty acid ester. In another embodiment, it is a fatty acid ester, which will tend to have a lower solubility in the glycol component (b) and may thus be more suitable - particularly if a strong solubilising agent such as Transcutol™ is included in the formulation - for achieving the desired saturation of the lipid. The fatty side chain of a component (c) may for example have from 8 to 28 carbon atoms, or (in particular for an unsaturated side chain) from 14 to 24 carbon atoms, or from 10 to 20 or 12 to 18 carbon atoms; it may be straight chain or branched, saturated or unsaturated. In an embodiment, it is a straight alkyl chain. In an embodiment, it is a saturated alkyl chain.

Suitable fatty acids include caprylic, nonanoic, capric, lauric, myristic, palmitic, heptadecanoic, stearic, arachidic, behenic, lignoceric, linolenic, linoleic, oleic, eicosapentaenoic, arachidonic, erucic and docosahexaenoic acids, and mixtures thereof. Suitable fatty alcohols include octanol, nonanol, 1-decanol, undecanol, dodecanol (lauryl alcohol), 1-tetradecanol, cetyl (palmityl) alcohol, stearyl alcohol, arachadic alcohol, doconasol, octanosol, oleyl alcohol, linolyl alcohol and mixtures thereof. Suitably fatty acid esters include octanoates, nonanoates, decanoates, laurates, myristates, palmitates, stearates, arachidates, docosates, octanoates, oleates, linoleates, and mixtures thereof. The ester may in particular be selected from isopropyl and propylene glycol esters, more particularly isopropyl esters, for example of C12 to C18 fatty acids.

In an embodiment, the fatty acid is selected from caprylic, nonanoic, capric, lauric, myristic, palmitic, stearic, arachidic, behenic, linolenic, linoleic, oleic, eicosapentaenoic, arachidonic, erucic and docosahexaenoic acids, and mixtures thereof. In an embodiment, the fatty alcohol is selected from octanol, dodecanol, 1-tetradecanol, cetyl alcohol, stearyl alcohol, arachadic alcohol, oleyl alcohol, linolyl alcohol and mixtures thereof; it may for example be dodecanol (lauryl alcohol). In an embodiment, the fatty acid ester is selected from octanoates, laurates, myristates, palmitates, stearates, arachidates, octanoates, oleates, linoleates, and mixtures thereof; or from myristates, palmitates and mixtures thereof.

In an embodiment, the component (c) is a palmitate, for example isopropyl palmitate (IPP). In an embodiment, it is a myristate, for example isopropyl myristate. In an embodiment, it is selected from isopropyl palmitate, isopropyl myristate, dodecanol and mixtures thereof. In another embodiment it is selected from isopropyl palmitate, isopropyl myristate and mixtures thereof. In a yet further embodiment it is isopropyl palmitate.

In an embodiment, it may be preferred for the component (c) not to be a fatty acid metal salt.

The alkylene glycol derivative (d), if present, may be an alkylene glycol ether. It may be an ethylene or propylene glycol derivative, in particular an ethylene glycol derivative. It may be a mono- or dialkylene glycol derivative (for example an ether), in particular a mono- or diethylene glycol derivative. In an embodiment it is a dialkylene glycol derivative.

Where the component (d) is an alkylene glycol ether, the ether component may for instance be a C1 to C6 ether, or a C1 to C4 ether, or a C1 to C3 ether, or a C2 to C4 ether.

Suitable alkylene glycol derivatives include ethylene glycol monomethyl ether, ethylene glycol monoethyl ether, ethylene glycol monopropyl ether, ethylene glycol monoisopropyl ether, ethylene glycol monobutyl ether, ethylene glycol monophenyl ether, ethylene glycol monobenzyl ether, diethylene glycol monomethyl ether, diethylene glycol monoethyl ether, diethylene glycol monopropyl ether, diethylene glycol mono-n-butyl ether, diisopropylene glycol ethyl ether and mixtures thereof.

The alkylene glycol derivative (d) may be selected from ethylene glycol monobutyl ether, diethylene glycol monoethyl ether (Transcutol™, a widely available penetration enhancer), diethylene glycol monomethyl ether, ethylene glycol monoethyl ether and mixtures thereof. In an embodiment it is selected from ethylene glycol monobutyl ether, Transcutol™, diethylene glycol monomethyl ether and mixtures thereof. In an embodiment, it is selected from ethylene glycol monobutyl ether, Transcutol™ and mixtures thereof. In an embodiment it is a diethylene glycol ether. It may in particular be Transcutol™.

The purpose of component (d) is to assist in solubilising the usnic acid or usnate active; it may also function as a partition coefficient enhancer, enhancing the transfer of the active from the formulation into the skin. Suitably, the component (d) has a polarity intermediate that of the glycol (b) and the hydrophobic component (c). Suitably, it is able to dissolve 1% w/w or greater of the usnic acid or usnate active, or 2 or 3 or 4 or 5% w/w or greater.

Each of the components (a) to (d) is suitably pharmaceutically acceptable, and/or acceptable for cosmetic use.

The concentration of the usnic acid or usnate in the formulation is suitably at least 0.05% w/w, or at least 0.1 or 0.5% w/w, or at least 0.8 or 1% w/w. It may be up to 5% w/w, or up to 4 or 3 or 2% w/w. In cases, for instance for "wash-off" formulations, it may be up to 10 or 9 or 8 or 7% w/w. In an embodiment, it is about 1% w/w. The nature and amount of the primary solvent or solvent system (a) - and if appropriate of the alkylene glycol derivative (d)- should ideally be chosen to ensure that the active is at or close to saturation in the mixture of components (a) to (d), as described above.

The concentration of the component (a) in the formulation may be 10% w/w or greater, or 12 or 14 or 15 or in cases 20 or 25 or 28 or 30% w/w or greater. It may be up to 60 or 50 or 40 or 35 or 30 or 25 or 20% w/w, for example from 10 to 60% w/w or from 20 to 50% w/w or from 30 to 40% w/w. However, where the component (a) is DMI, its concentration in the formulation should be up to 40% w/w or up to 35 or 30% w/w; it may for example be from 5 to 40% w/w or greater, or from 5 to 35% w/w, or from 10 to 40 or 35% w/w.

The concentration of the glycol (b) in the formulation may be 10% w/w or greater, or 20 or 30% w/w or greater, or 35 or 40% w/w or greater. It may be up to 60 or 55 or 50 or 45 or 40% w/w, for example from 10 to 60% w/w or from 20 to 50% w/w or from 30 to 40% w/w.

The concentration of the component (c) in the formulation may be 1% w/w or greater, or 3 or 3.5 or 4 or 4.5 or 5 or 6% w/w or greater. It may be up to 15 or 10 or 8 or 6 or 5% w/w, for example from 1 to 15% w/w or from 5 to 10% w/w or from 6 to 8% w/w.

If present, the concentration of the alkylene glycol derivative (d) in the formulation may be 10% w/w or greater, or 15 or 20 or 25 or 30 or 35% w/w or greater. It may be up to 60 or 50 or 40 or 35 or 30 or 25 or 20% w/w, for example from 10 to 60% w/w or from 20 to 50% w/w or from 30 to 40% w/w.

In an embodiment, the combined concentrations of the components (b) to (d) in the formulation total greater than 40% w/w.

For any chosen set of components (a) to (d), suitable concentration ratios may be determined from phase diagrams, ensuring that, as described above, both the active and the hydrophobic component are present at or close to their saturation levels. Ideally the ratios are also chosen so that the active concentration is at an antimicrobially effective level in the resultant formulation. Each component and its relative concentration can suitably be chosen so as to optimise its likely delivery rate on topical application, and thus to increase the synergy between components in enhancing delivery of the usnic acid or usnate active.

The components (a) to (d) may be combined with other standard excipients, which may be chosen so as to give the formulation a desired physical form. The formulation may for example include one or more surfactants. It may contain one or more thickeners or gelling agents. In particular if it is to be formulated as a cream, it may include one or more surfactants and one or more waxes. In particular if it is to be formulated as a spray or gel, it may include a volatile solvent and a thickening polymer. It may be formulated as a skin patch, in which a suitable substrate is impregnated or coated with the formulation.

A thickener or gelling agent, if present, may be a cellulose-based thickening agent such as ethyl cellulose, hydroxyethyl cellulose, hydroxypropyl cellulose, hydroxypropyl methyl cellulose or a carboxymethyl cellulose. Such agents may be used in the form of a (preferably pharmaceutically acceptable) salt such as for instance the sodium salt. The thickening agent may be a polymeric thickening agent such as a carbomer, which will typically be a homopolymer of acrylic acid, cross-linked with an allyl ether. In an embodiment, the thickening agent is a cellulose-based thickener, in particular hydroxypropylcellulose (HPC) which is available for example as Klucel™. A thickener or gelling agent may be present in the formulation at a concentration of 0.2% w/w or greater, or 0.5 or 0.75 or 1% w/w or greater. It may be present at a concentration of up to 5 or 4 or 3 or 2% w/w, for example from 0.5 to 5% w/w or from 0.75 to 3% w/w or from 1 to 2% w/w, such as about 2% w/w.

A formulation according to the invention contains, as an active agent, either usnic acid or an usnate salt. Usnic acid may be used in any one of its tautomeric forms. It may be used in the form of its d- or 1-isomer, or of a racemic mixture. The same applies, *mutatis mutandis,* to usnate salts.

An usnate salt is suitably a pharmaceutically acceptable and/or cosmetically acceptable usnate. It may for example be a metal salt. It may be an alkali metal salt such as sodium usnate. It may be copper usnate (typically copper (II) usnate).

An usnate may be used in the form of a solvate such as a hydrate. It may be in the form of an oligomer or polymer, for example in which metal ions bind to more than one site in the usnate ion (see for example Takani M et al, 2002, Journal of Inorganic Biochemistry 91: 139-150). It may be either synthetic or naturally occurring (for example it may be used in the form of an extract from a plant source such as *Usneaceae*, *Evernia, Parmelia* or *Cladonia*), as may usnic acid itself.

The usnic acid or usnate may be used in the form of a derivative. In general a "derivative" may be a pharmaceutically acceptable (which term includes acceptable for veterinary use) derivative. It may be for example a salt, complex or solvate or a so-called "prodrug" form or protected form which reverts to an active form of the relevant compound at an appropriate time on or after administration. In an embodiment of the invention, however, the Usnic acid or usnate is present in the form of an underivatised usnic acid molecule or usnate ion as the case may be.

In an embodiment of the invention, the usnic acid or usnate active is selected from usnic acid, sodium usnate, copper usnate and mixtures thereof. In an embodiment, it is selected from usnates (in particular metal usnates) and mixtures thereof, in particular sodium and copper usnates and mixtures thereof. In an embodiment, it is copper usnate.

In an embodiment, it may be preferred for the formulation not to contain DispersinB™. In an embodiment, it may be preferred for the formulation not to contain an extract of St John's Wort. In an embodiment, it may be preferred for the formulation not to contain resveratrol. In an embodiment, it may be preferred for the formulation not to take the form of an oil-in-water emulsion.

The formulation of the invention contains 1% w/w or less of water, in order to reduce the risk of phase separation amongst the remaining components.

In a formulation according to the invention, the usnic acid or usnate may be present as an antimicrobially, typically antibacterially, active agent. It may be present as an anti-acne agent (ie as an agent which is active against acne (which includes against a symptom and/or a cause of acne and/or against one or more micro-organisms associated with acne)).

In the present context, antimicrobial activity embraces activity against bacteria, fungi, viruses and other micro-organisms, in particular against bacteria. It may be growth inhibitory activity or more preferably biocidal (ie lethal to the relevant organism). Antibacterial activity encompasses activity against both Gram-positive and anaerobic Gram-negative bacteria, and it may comprise activity against sessile and/or planktonic bacteria. In the context of this invention, activity against a particular species of micro-organism may be taken to mean activity against at least one, preferably two or more, strains of that species.

Antimicrobial activity may be or include the ability to disrupt and/or suppress biofilm formation by the relevant organism. The disruption of biofilm formation embraces any negative effect on the ability of a micro-organism to form, maintain or exist in a biofilm, and/or on a biofilm already formed by the organism. Thus, it may involve reducing the amount of a previously formed biofilm, and/or impairing such a biofilm. It may involve killing or inhibiting sessile micro-organisms within a biofilm. Suppression of biofilm formation embraces any degree of impairment (including complete prevention) of the ability of a micro-organism to form, or more typically to co-aggregate with, a biofilm. It thus embraces total or partial impairment, including reducing the amount and/or strength of biofilm which the organism is able to form and/or the speed with which it is able to do so. It may involve preventing or reducing the growth or the rate of growth of an existing biofilm formed by the organism.

A formulation according to the present invention may be active, suitably as a bactericide, against Gram-positive and/or Gram-negative bacteria, in particular Gram-positive bacteria. It may be active against one or more propionibacteria. In particular, it may be active against one or more bacteria associated with acne, in particular propionibacteria such as *P. acnes* and in some instances *P. granulosum.* It may be active against one or more staphylococci, such as *S. aureus.* It may be active against one or more bacteria implicated in body odour, in particular in the axilla or feet.

The formulation may be active against micro-organisms, in particular bacteria and more particularly propionibacteria and/or staphylococci, which are wholly or partially resistant to one or more antibiotics, for instance those which are in common clinical use. For example it may be active against one or more macrolide-lincosamide-streptogramin (MLS) resistant and/or macrolide-lincosamide-streptogramin-ketolide (MLSK) resistant strains of bacteria. It may be active against MRSA bacterial strains, and/or against other resistant staphylococci such as VISA or GISA. It may be active against one or more erythromycin-resistant, clindamycin-resistant and/or tetracycline-resistant strains of bacteria, for example *P. acnes* strains, the term tetracycline here referring to the class of antibiotics including for example minocycline and doxycycline as well as the specific antibiotic known as tetracycline.

The formulation of the invention is preferably suitable for topical application to the skin. It may be suitable for topical application to the scalp and/or eyes, in particular the eyes. It may be suitable for topical application to tissue surfaces within the nares and/or ears, in particular the nares. It may in particular be suitable for topical application to such surfaces in or on the human body. A formulation which is "suitable for" topical application may also be adapted for topical application.

A formulation according to the invention may take the form of a lotion, cream, ointment, varnish, foam, paste, gel or any other physical form known for topical administration. It may take the form of a solution or emulsion, in particular the former. It may take the form of a spray or aerosol (for example for nasal or oral use), or of drops (for example for use in the eyes or ears). It may comprise a formulation which is, or may be, applied to a carrier such as a sponge, swab, brush, pad, tissue, cloth, wipe, skin patch or dressing (which includes a bandage, plaster, skin adhesive or other material designed for application to a tissue surface, in particular to a wound) to facilitate its topical administration. It may be intended for pharmaceutical (which includes veterinary but is preferably human) use, and/or for cosmetic or other non-medical care purposes (for example, for general hygiene or skin cleansing or for improving the appearance of the skin).

The formulation is in the form of a liquid, for example a lotion, cream, ointment, varnish, foam, paste, gel or other viscous or semi-viscous liquid, or a less viscous liquid such as might be used in sprays, drops and dropping fluids, or aerosols. A liquid formulation may itself be formulated as suspended (for example aerosolised) liquid droplets within another fluid carrier.

As described above, the formulation may contain, in addition to the components (a) to (d), one or more conventional solvents, excipients or other additives which are suitable for topical or local application. Where the formulation is intended for topical application to the skin, in particular to treat acne, examples of suitable additives include emollients, perfumes, antioxidants, preservatives, stabilisers, gelling agents and surfactants. For the treatment of acne, however, it may be preferred for the formulation not to contain an emollient.

Such a formulation may further contain additional active agents such as antimicrobial (in particular antibacterial) agents. For example, it may contain one or more agents selected from anti-acne agents, keratolytics, comedolytics, agents capable of normalising keratinocyte and/or sebocyte function, anti-inflammatories, anti-proliferatives, antibiotics, anti-androgens, sebostatic/sebosuppressive agents, anti-pruritics, immunomodulators, agents which promote wound healing, additional antimicrobial agents, and mixtures thereof; it may instead or in addition contain one or more agents selected from sunscreens, moisturisers, emollients and mixtures thereof.

An additional antimicrobial agent may be selected from biocides, disinfectants, antiseptics, antibiotics, bacteriophages, enzymes, anti-adhesins, immunoglobulins, antimicrobially active antioxidants, and mixtures thereof; it may be active as a bactericide, in particular against staphylococci and/or propionibacteria. It may however be preferred for the usnic acid or usnate to be the only active agent in the formulation, or at least to be the only antimicrobially or antibacterially active agent and/or the only anti-acne active agent.

The formulation may contain one or more agents which target or control release of the active ingredient(s) at the intended site of administration, for instance to target a desired site and/or time of delivery. Such agents may for instance target the active(s) to the skin or hair follicles, or to the anterior nares (the latter being particularly suitable when the formulation is used as a preventative treatment against staphylococci). They may delay or otherwise control release of the active(s) over a particular time period. The formulation may contain one or more surface-active agents, in particular when it is intended for use in treating surfaces, for instance to cleanse instruments or working areas in particular against staphylococci.

Where the formulation is intended for use in the treatment of an ocular infection, it may take the form of a cream or ointment, or of eye drops, or of an eye rinse. Such formulations are typically aqueous based. Where the formulation is intended for use in the treatment of body odour, it may contain an anti-perspirant such as an aluminium or aluminium-zirconium salt. It may be in the form of an aerosol, or of a roll-on or "stick" deodorant of known type, containing appropriate conventional liquid or solid carriers and excipients. It may contain one or more perfumes.

A formulation according to the invention may be incorporated into, and hence applied in the form of, another product such as a cosmetic, a skin or hair care preparation (for example a skin cleanser, toner or moisturiser, or a shampoo, conditioner, styling mousse or gel or hair spray), a deodorant or anti-perspirant, a cleansing preparation (for example a hand wash or facial wash), a pharmaceutical (which includes veterinary) preparation, a cosmeceutical preparation, a toiletry product (for instance a bath or shower additive or a soap), or a laundry or other fabric treatment product. The formulation may be, or be incorporated into, a leave on or a wash off skin treatment product.

The invention thus provides, according to a second aspect, a product which incorporates an antimicrobial and/or anti-acne formulation according to the first aspect.

A formulation according to the invention may be marketed with an indication that it has antimicrobial (in particular antibacterial) and/or anti-acne activity, or enhanced antimicrobial and/or anti-acne activity. The marketing of such a formulation may include an activity selected from (i) enclosing the formulation in a container or package that comprises the relevant indication; (ii) packaging the formulation with a package insert that comprises the indication; (iii) providing the indication in a publication that describes the formulation; and (iv) providing the indication in a commercial which is aired for instance on the radio, television or internet. The activity may be attributed, in such an indication, at least partly to the presence of the usnic acid or usnate, and/or to that of one or more of the components (a) to (d). The invention may involve assessing the activity of the formulation during or after its preparation, for instance against one or more of the pathogens referred to below. It may involve assessing the activity both before and after incorporation of the usnic acid or usnate or any one of the components (a) to (d), for example so as to confirm that the relevant component contributes to the antimicrobial or anti-acne activity of the formulation.

Also disclosed is a method for preparing an antimicrobial or anti-acne formulation, which method involves mixing together usnic acid or an usnate, and the components (a) to (c) as defined above, optionally with component (d). In an embodiment, the usnic acid or usnate is at least partially solubilised in the primary solvent or solvent system (a), together with the component (d) if appropriate, and then added to components (b) and (c). Components (b) and (c) may be premixed, or may be added sequentially to the solubilised active, for instance component (b) followed by component (c). In an alternative embodiment, the usnic acid or usnate is at least partially solubilised in a mixture of the components (a), (b) and if appropriate (d), followed by the addition of component (c).

After the usnic acid or usnate has been added to component (a) (and where appropriate component (d)), it may be necessary to filter the mixture to remove undissolved active.

Once the usnic acid or usnate has been mixed with the components (a) to (d), a thickener or gelling agent may be added.

According to a third aspect of the invention there is provided a formulation according to the first aspect, for use in the treatment of a condition affecting the human or animal body, which condition is caused by, transmitted by and/or exacerbated by (in particular either caused or transmitted by) microbial activity. The activity may in particular be bacterial activity, more particularly staphylococcal or propionibacterial activity.

In the context of the present invention, treatment of a condition encompasses both therapeutic and prophylactic treatment, of either an infectious or a non-infectious condition, in either a human or animal but in particular a human. It may involve complete or partial eradication of the condition, removal or amelioration of associated symptoms, arresting subsequent development of the condition, and/or prevention of, or reduction of risk of, subsequent occurrence of the condition. It will typically involve use of the usnic acid or usnate as an antimicrobial and/or anti-acne agent. It may involve the prophylactic treatment of any area of the body, in particular the skin or nares or ears or another epithelial or mucosal surface, against microbial infections, including against staphylococcal infections such as those associated with MRSA.

The treatment of a condition also embraces the prevention, or reduction of risk of, dissemination or transmission of the condition, for example person to person. In this context, the formulation of the invention may be used as a disinfectant against the relevant micro-organism, for example for antisepsis of the skin and/or other appropriate parts of the body, or for the general disinfection of surfaces in an area believed to be contaminated with, or at risk of contamination with, the organism. It may be used to treat an outbreak of a particular pathogen, for example a nosocomial pathogen such as S. aureus (including resistant strains such as MRSA, VISA or GISA).

Treatment may involve use of the formulation to treat a condition which is caused, transmitted and/or exacerbated by (in particular caused or transmitted by) microbial biofilm formation. The biofilm may in particular be formed by a bacterium, such as Propionibacterium acnes.

The treatment will typically be administered topically or locally.

In an embodiment of the third aspect of the invention, the formulation is for use against one or more bacteria associated with skin or skin-borne infections. It may be for use against Gram-positive bacteria, for example staphylococci and/or propionibacteria, in particular against strains of Staphylococcus aureus and/or Propionibacterium acnes. In an embodiment, it is for use against one or more bacteria associated with acne, such as P. acnes and in some instances P. granulosum.

Thus in an embodiment, the formulation is for use in the treatment of a skin or skin structure condition. Such conditions include acne, infected atopic eczema, superficial infected traumatic lesions, soft tissue infections, wounds, burns, ulcers, folliculitis, mycoses, boils, carbuncles, impetigo, erysipelas, erythrasma, cellulitis, infected dermatoses, and other primary and secondary skin and skin structure infections. The condition may for example be a superficial or uncomplicated skin infection amenable to local therapy. It may be acne or an infection associated with. It may be a primary or secondary infection (for example infected atopic dennatitis) due to *S. aureus* (including MRSA) or a group A beta haemolytic streptococcus such as *S. pyogenes.* In particular the formulation may be for use in treating acne or acne lesions (for instance, to reduce acne-related scarring).

Acne is a multifactorial disease of the pilosebaceous follicles of the face and upper trunk, characterised by a variety of inflamed and non-inflamed lesions such as papules, pustules, nodules and open and closed comedones. Its treatment can therefore encompass the treatment (which embraces prevention or reduction) of any of these symptoms, and references to use as an anti-acne agent may be construed accordingly. In particular, the treatment of acne encompasses the treatment (including prevention) of lesions and/or scarring associated with acne. It also encompasses the inhibition of propionibacterial activity which could cause or be otherwise associated with acne or its symptoms. In the context of the present invention, it may in particular be the treatment of inflamed acne lesions.

In general, the present invention will be used for the treatment of symptoms which are directly due to acne rather than for instance infections which may arise as a consequence of treating acne with other actives such as antibiotics, and/or secondary infections caused by opportunistic pathogens, which can arise in skin already affected by acne. It will not generally be used for the treatment of symptoms which are acne-like but which are not etiologically the same as acne, for instance skin rashes caused by treatment with other medicaments such as epidermal growth factor receptor (EGFR) inhibitors.

Instead or in addition, the formulation may be for use against an opportunistic infection which is caused, transmitted and/or exacerbated by (in particular caused by) propionibacteria, for instance an infection associated with an indwelling surgical device (a prosthetic joint or catheter, for example). It may be for use in treating an infected wound, burn or ulcer. It may be for use against any other infection or condition which involves or can involve propionibacteria, in particular an eye infection such as endophthalmitis, or in cases body odour.

In an embodiment, the formulation of the first aspect of the invention may be for use in the treatment of a staphylococcal infection, for instance on the skin, or in the nares, eyes or ears. It may in particular be for use in a prophylactic treatment against staphylococci (in particular *S. aureus*) in the nasal carriage.

According to a further embodiment, the formulation may be for use in the treatment (which includes prevention) of body odour, in particular human body odour, for example in the axilla or feet. It may thus be for use against one or more bacteria implicated in this condition, in particular aerobic diphtheroids of the genus *Corynebacterium,* and/or other members of the bacterial human skin microflora such as cutaneous propionibacteria or coagulase negative staphylococci.

In an embodiment, the formulation may be for use in the treatment of an ocular infection. It may for example be used in the treatment of conjunctivitis due to *Corynebacterium* spp or in particular *S. aureus,* or in the treatment (in particular the prevention) of endophthalmitis due to *Propionibacterium* spp.

In an embodiment, the formulation may be for use in the treatment of an infection associated with an indwelling surgical device, prosthesis or implant, for example a catheter. Such an infection may for instance be due to *Staphylococcus aureus,* coagulase-negative staphylococci, streptococci or cutaneous propionibacteria. It may be linked to (ie may in cases cause, increase susceptibility to and/or exacerbate) other, systemic conditions such as bacteraemia and its sequlae, including for example acute and subacute endocarditis. Thus the formulation may be for use in the treatment of such infections and/or associated conditions.

In an embodiment, the formulation is for use in the treatment of an infected dermatosis, a skin infection, a superficial infected wound or burn or a soft tissue infection. Such conditions can include for example folliculitis, boils and carbuncles, impetigo most usually caused by *Staphylococcus aureus* and other infections including erysipelas caused by members of the genus *Streptococcus,* erythrasma and cellulitis caused by both staphylococci and streptococci.

In an embodiment, it may be for use in the disinfection of skin or other tissue surfaces. It may in particular be used to counter bacteria of the type referred to above, for example S. aureus.

In an embodiment, the formulation is for use against one or more bacteria selected from staphylococci (in particular S. aureus and in cases also coagulase-negative staphylococci such as S. auricularis, S. capitis, S. cohnii, S. epidermidis, S. haemolyticus, S. hominis, S. saprophyticus, S. simulans, S. warneri and S. xylosus), cutaneous propionibacteria (in particular P. acnes) and members of the genus Corynebacterium. In an embodiment, it is for use in the treatment of a condition selected from acne, body odour and staphylococcal infections.

Also disclosed is the use of a formulation according to the first aspect in the manufacture of a medicament (typically a formulation) for the treatment of a condition which is caused by, transmitted by and/or exacerbated by (in particular either caused or transmitted by) microbial activity. The condition may be selected from those listed above in connection with the first to the third aspects of the invention. It may in particular be acne. It may be a staphylococcal infection. The usnic acid or usnate will typically be used as an antimicrobial and/or anti-acne agent in the manufacture of the medicament.

Also disclosed is the use of a formulation according to the first aspect for non-therapeutic purposes. In an embodiment of this aspect, the formulation is used as an anti-acne or skin care agent for non-therapeutic purposes, for example for cosmetic purposes such as to improve the appearance, feel or smell of the skin.

Also disclosed is a method for controlling the growth of a micro-organism, the method comprising applying, to an area or surface which is infected or suspected to be infected or capable of becoming infected with the organism, a formulation according to the first aspect of the invention. The formulation is suitably applied topically. It may in particular be applied to an area or surface which is infected with the relevant micro-organism. The organism may be a bacterium, for example a propionibacterium or a staphylococcus.

"Controlling the growth" of an organism embraces inhibiting or preventing its growth, whether completely or partially, as well as killing either completely or partially a culture of the organism. It also embraces reducing the risk of subsequent growth of the organism in or on the area or surface being treated. It may embrace reducing the risk of transmission of the organism from the area or surface being treated to another area or surface and/or living body. The method of the invention may thus be used to treat an existing occurrence of the organism or to prevent a potential subsequent occurrence. Controlling the growth of a micro-organism may also embrace the disruption and/or suppression of biofilm formation by the organism.

Where the formulation is applied to living human or animal tissue, it may be applied for therapeutic purposes or for non-therapeutic (eg purely cosmetic) purposes. Thus this aspect of the invention encompasses a method of treatment of a human or animal patient suffering from or at risk of suffering from a condition which is caused by, transmitted by and/or exacerbated by (in particular either caused or transmitted by) microbial activity, the method involving administering to the patient a therapeutically (which term includes prophylactically) effective amount of a formulation according to the first aspect of the invention. The condition may be any of those referred to in connection with the first to the third aspects, for example acne or a staphylococcal infection. The formulation is suitably administered in an antimicrobially effective amount. It is suitably administered topically.

In accordance with this aspect of the invention, the formulation is suitably administered to a human patient. The patient is suitably suffering from the relevant condition.

Throughout the description and claims of this specification, the words "comprise" and "contain" and variations of the words, for example "comprising" and "comprises", mean "including but not limited to", and do not exclude other moieties, additives, components, integers or steps. Moreover the singular encompasses the plural unless the context otherwise requires: in particular, where the indefinite article is used, the specification is to be understood as contemplating plurality as well as singularity, unless the context requires otherwise.

Preferred features of each aspect of the invention may be as described in connection with any of the other aspects. Other features of the invention will become apparent from the following examples.

References to solubilities are, unless otherwise stated, to measurements at standard temperature and pressure.

The present invention will now be further described with reference to the following non-limiting examples.

### Detailed description

### Example 1

A gel formulation according to the invention, with a volatile primary solvent base, was prepared using the ingredients listed in Table 1 below, in the proportions shown.

**Table 1**

| ***Ingredient*** | ***Function*** | ***% w*/*w*** |
|---|---|---|
| Copper usnate | Active | 1.00 |
| Transculol™ | Partition coefficient co-enhancer | 36.95 |
| Propylene glycol | Partition coefficient co-enhancer | 36.95 |
| Isopropyl palmitate | Diffusion coefficient co-enhancer | 4.50 |
| Acetone | Volatile solvent | 18.60 |
| Klucel™ HF | Gelation agent | 2.00 |
| Total | | 100.00 |

To prepare this gel, copper usnate was dissolved in the mixed solvents acetone, Transcutol™ and propylene glycol. The isopropyl palmitate was then added, and the mixture filtered to remove any undissolved usnate. To the resulting miscible solution Klucel™ HF was added and mixed with low shear; the mixture was then left overnight to solvate and form a clear gel.

### Example 2

Gel formulations A and B, both containing DMI as the primary solvent, can be prepared using the ingredients listed in Table 2 below. Component concentrations are shown in % w/w.

**Table 2**

| ***Ingredient*** | ***Function*** | ***Formulation A (% w*/*w)*** | ***Formulation B (% w*/*w)*** |
|---|---|---|---|
| Copper usnate | Active | 1.00 | 1.00 |
| Transcutol™ | Partition coefficient co-enhancer | 31.53 | 32.00 |
| Propylene glycol | Partition coefficient co-enhancer | 45.06 | 45.10 |
| Isopropyl palmitate | Diffusion coefficient co-enhancer | 5.41 | 4.9 |
| DMI | Non-volatile solvent; partition coefficient co-enhancer | 15.00 | 15.00 |
| Klucel™ HF | Gelation agent | 2.00 | 2.00 |
| Total | | 100.00 | 100.00 |

These gels can be prepared using an analogous method to that of Example 1, the copper usnate initially being dissolved in the mixed solvents DMI, Transcutol™ and propylene glycol. Again, both the copper usnate and the isopropyl palmitate are present at, or close to, saturation in the overall formulations.

### Example 3

A third DMI-based gel formulation was prepared using the ingredients listed in Table 3 below.

**Table 3**

| ***Ingredient*** | ***Function*** | **% *w*/*w*** |
|---|---|---|
| Copper usnate | Active | 1.00 |
| Transcutol™ | Partition coefficient co-enhancer | 17.63 |
| Propylene glycol | Partition coefficient co-enhancer | 44.08 |
| Isopropyl palmitate | Diffusion coefficient co-enhancer | 5.29 |
| DMI | Non-volatile solvent; partition coefficient co-enhancer | 30.00 |
| Klucel™ HF | Gelation agent | 2.00 |
| Total | | 100.00 |

This gel was prepared using the same method as that of Example 2. The copper usnate and the isopropyl palmitate were present at, or close to, saturation levels.

The formulations of Examples 1 to 3 are all suitable for topical application to the skin, for instance as antimicrobial and/or anti-acne agents. In all four cases, a therapeutically effective dose of the usnate active can be successfully solubilised, despite the relatively poor solubility of this compound in most common solvents. Moreover the inclusion of the glycol, Transcutol™ and palmitate can significantly enhance the rate at which the active partitions out of the formulation and diffuses into the skin, following topical application, thus significantly increasing the amount of the usnate which can reach the target site.

### Example 4

Alternative gel formulations 4A to 4J, in accordance with the invention, were prepared using the ingredients listed in Table 4 below. The figures quoted in the table are percentages by weight for the various ingredients of the formulations.

**Table 4**

| ***Ingredient*** | ***4A*** | ***4B*** | ***4C*** | ***4D*** | ***4E*** | ***4F*** | ***4G*** | ***4H*** | ***4I*** | ***4J*** |
|---|---|---|---|---|---|---|---|---|---|---|
| Copper usnate | 1 | 1 | 1 | 1 | 1 | 1 | 1 | 1 | 1 | 1 |
| Transcutol™ | 32 | 32 | 32 | 32 | 32 | 0 | 0 | 0 | 32 | 32 |
| Ethylene glycol monobutyl ether | 0 | 0 | 0 | 0 | 0 | 32 | 0 | 0 | 0 | 0 |
| Diethylene glycol monomethyl ether | 0 | 0 | 0 | 0 | 0 | 0 | 32 | 0 | 0 | 0 |
| Ethylene glycol monoethyl ether | 0 | 0 | 0 | 0 | 0 | 0 | 0 | 32 | 0 | 0 |
| Propylene glycol | 5.1 | 0 | 0 | 45.1 | 45.1 | 45.1 | . 45.1 | 45.1 | 45.1 | 45.1 |
| Dipropylene glycol | 0 | 45.1 | 0 | 0 | 0 | 0 | 0 | 0 | 0 | 0 |
| 1,3-butanediol | 0 | 0 | 45.1 | 0 | 0 | 0 | 0 | 0 | 0 | 0 |
| Isopropyl palmitate | 4.9 | 4.9 | 4.9 | 0 | 0 | 4.9 | 4.9 | 4.9 | 4.9 | 4.9 |
| Isopropyl myristate | 0 | 0 | 0 | 4.9 | 0 | 0 | 0 | 0 | 0 | 0 |
| Lauryl alcohol | 0 | 0 | 0 | 0 | 4.9 | 0 | 0 | 0 | 0 | 0 |
| DMI | 0 | 15 | 15 | 15 | 15 | 15 | 15 | 15 | 0 | 0 |
| Butanone | 15 | 0 | 0 | 0 | 0 | 0 | 0 | 0 | 0 | 0 |
| Phenoxyethanol | 0 | 0 | 0 | 0 | 0 | 0 | 0 | 0 | 15 | 0 |
| THFA | 0 | 0 | 0 | 0 | 0 | 0 | 0 | 0 | 0 | 15 |
| Klucel™ HF | 2 | 2 | 2 | 2 | 2 | 2 | 2 | 2 | 2 | 2 |
| Total | 100 | 100 | 100 | 100 | 100 | 100 | 100 | 100 | 100 | 100 |

In Examples 4A, 4I and 4J, butanone, phenoxyethanol and THFA respectively were used as primary solvents for the copper usnate, in place of the DMI used in Examples 2A and 2B. In Examples 4B and 4C, dipropylene glycol and 1,3-butanediol were used as glycol components (b). In Examples 4D and 4E, isopropyl myristate and lauryl alcohol were used as hydrophobic components (c). In Examples 4F, 4G and 4H, ethylene glycol monobutyl ether, diethylene glycol monomethyl ether and ethylene glycol monoethyl ether were used as alkylene glycol derivatives (d).

These gels were prepared using an analogous method to that of Example 1. The copper usnate was initially dissolved in the mixture of solvent components (a) and (d), prior to addition of component (b), then (c), and finally the Klucel™ thickener. In each of the formulations, the copper usnate was present as a saturated solution in the remaining components. Moreover in each case, the hydrophobic component (c) (isopropyl palmitate, isopropyl myristate or lauryl alcohol) was also present at or close to saturation in the overall formulation.

Of the formulations 4A to 4J, those showing the best miscibility of solvent phases were 4A, 4B, 4C, 4D, 4E, 4F, 4G and 4H. Those showing particularly good phase miscibility were 4A, 4B, 4D, 4F and 4G.

### Example 5

An alternative formulation according to the invention was prepared using the same ingredients and concentrations as Example 2B, but with 1% w/w sodium usnate in place of the 1% w/w copper usnate. Again, the result was a gel formulation suitable for topical application to the skin.

### Example 6 to 13

Experimental tests were conducted to determine the antimicrobial and anti-acne activity of formulations according to the invention.

### Test micro-organisms

The first test micro-organism used was *Propionibacterium acnes* NCTC 737. This is a propionibacterial strain and is the type strain of the genus; it is fully susceptible to antibiotics.

The propionibacteria are clinically significant due to their involvement in acne. This is a very common, complex and multi-factorial skin disease in which *P. acnes* and other *Propionibacterium* spp. (for example *P. granulosum*) play key roles. They are also opportunistic pathogens in compromised hosts. Thus, activity observed against these micro-organisms is expected to be a good predictor of activity against acne.

The propionibacteria were cultured and maintained on Wilkins-Chalgren Anaerobe Medium (agar and broth) at pH 6.0; all cultures were incubated anaerobically at 37°C for 72 hours.

The other pathogens tested were:
(a) staphylococci. The *Staphylococcus aureus* strain used was ATCC 29213. S. *aureus* and other staphylococci are common causes of a wide range of skin, skin structure and wound infections; *S. aureus* is also known to exacerbate eczema. The ATCC 29213 strain is known to be susceptible to beta-lactam antibiotics such as methicillin. The *Staphylococcus epidermidis* strain used was NCTC 11047. *Staph. epidermidis* forms part of the resident flora of skin and is an opportunistic pathogen which can cause biofilms to grow for instance on plastic surgical implants such as catheters. Unless otherwise stated, the staphylococci were cultured and maintained on Mueller-Hinton Medium (agar and broth) at pH 7.2; cultures were incubated aerobically at 37°C for 19-20 hours, at pH 7 (± 0.2).
(b) *C. striatum* NCTC 764. *Corynebacterium striatum* is closely related to organisms (aerobic diphtheroids of the genus *Corynebacterium*) that cause body odour. It was cultured and maintained on Mueller-Hinton medium (agar and broth). All cultures were incubated aerobically at 37°C for 72 hours, at pH 7 (± 0.2).
(c) streptococci. The strains tested were *S. mutans* ATCC 25175 and *S. pyogenes* ATCC 12344. *S. mutans* is a Gram-positive, microaerophilic bacterium which plays a significant role in dental caries and in infective endocarditis. *Streptococcus pyogenes* is associated with skin, soft tissue, burn and wound infections. *S. mutans* was cultured and maintained on Wilkins-Chalgren Anaerobe medium + 1g/L glucose (agar and broth); it was incubated in air at 37°C for 48 hours, at pH 7 (± 0.2) with 5% carbon dioxide. *S. pyogenes* was cultured and maintained on Mueller-Hinton medium (broth and agar) and was incubated under the same conditions as *S. mutans.*

The following tests were carried out to assess antimicrobial activity against the test organisms.

### (a) Minium inhibitory concentration (MIC) assay

This is a standard international method for quantitatively assessing the antimicrobial activity of a compound in a liquid medium. The method used a sterile 96-well microtitre plate, capable of holding about 200 µl of liquid per well. The wells contained liquid culture medium and ranges of decreasing concentrations of the relevant test compound in doubling dilutions (eg 1000, 500, 250, 125...µg/ml, etc.. down to 0.49 µg/ml). The culture medium was as described above.

The wells were inoculated with a liquid suspension of freshly grown micro-organism and incubated under the conditions described above. After incubation, the microtitre plate was examined visually (with the aid of a light box) for cloudiness in each well, which would indicate microbial growth. The MIC value was recorded as the lowest concentration of test compound required to inhibit microbial growth, ie the lowest concentration for which the liquid in the well remained clear.

The assays included both negative (culture medium with no micro-organisms) and positive (culture medium plus diluting solvent plus micro-organism) controls.

Since inhibition does not necessarily indicate killing of microbial cells, merely that growth as visible to the naked eye has been inhibited, it is desirable to conduct a further test (the MBC assay described below) to establish the concentration of the test compound needed to kill the test organism.

### (b) Minimum bactericidal concentration (MBC) assay

This assay, normally carried out after an MIC assay, determines the minimum concentration of a compound that is lethal to the micro-organism being tested.

Following an MIC assay, a 5 µl sample was withdrawn from the first microtitre well that showed positive growth and from all the subsequent wells that showed no growth. These samples were then individually sub-cultured on antibiotic-free agar medium, under the incubation conditions described above. Following incubation they were examined visually for microbial growth. The MBC was taken to be the lowest test compound concentration for which the incubated sample showed no growth.

The ratio of MIC to MBC should ideally be as close to 1 as possible. This facilitates selection of the lowest possible effective concentration of a test compound with a reduced risk of selecting a sub-lethal concentration which could promote resistance or allow the target microbial population to recover.

### (c) Agar dilution MIC assay

This is a standard international method for quantitatively assessing the antimicrobial activity of a compound in a solid medium. The test compound was prepared to 40× the highest concentration required (eg 10 mg/ml for a final concentration of 250 µg/ml) and a series of doubling dilutions were performed in a suitable solvent. A set amount of these antimicrobial stock solutions was then added to molten agar medium (ca 55°C), mixed thoroughly, poured into sterile Petri dishes and allowed to cool/set. The culture medium was as described above.

A Multipoint™ Inoculator (AQS Manufacturing Ltd, UK) was used to inoculate the plates by spotting the inocula onto the surface of the agar, delivering approximately 1 to 2 µl per spot (yielding 10⁵ CFU (colony forming units) per spot).

The plate(s) were then incubated under the conditions described above, following which they were examined visually for signs of bacterial growth. The MIC value was ascertained when there was a marked reduction in, or total loss of, growth on the test plate at the lowest concentration as compared to that of the growth on the control plate.

The assays were conducted in triplicate and included a positive control (culture medium, diluting solvent and inoculum).

### (d) Disc Diffusion assay (DDA)

This is an internationally recognised standard method for qualitatively assessing the antimicrobial activity of a compound.

A sterile paper disc was impregnated with a sample of the test compound in a suitable solvent and 30 minutes allowed for the solvents to evaporate (where possible). The disc was then placed on an agar plate onto which the test micro-organism had been inoculated. The plate was then incubated under the conditions described above, following which it was examined visually for signs of microbial growth. If the test compound had antimicrobial activity, a circular zone of no growth would be obtained around the disc. The diameter of this zone of "inhibition" was measured using a ProtoCOL™ automated zone sizer (Synbiosis, Cambridge, UK). In general, a greater diameter and/or area of the zone of inhibition indicates a greater antimicrobial activity in the relevant test compound, although other factors such as test compound mobility through the agar gel may also influence the result.

### (e) Supplemented disc diffusion assay

The DDA test may be carried out using an agar gel supplemented with lipid and/or salt to simulate some of the major components present in human skin and to assess whether these substances might affect the antimicrobial activity observed for the test compound. Performance under these conditions can provide a more reliable indication of activity on topical application. The supplements used in Example 6 below were lipid (triolein at 1% v/v) and sodium chloride (100 mM).

### Example 6 - in vivo antimicrobial activity

The formulation of Example 2B was topically applied, twice daily, to three locations on the face of a healthy human volunteer. The skin was sampled before the treatment began, and at 2, 7, 10 and 14 days afterwards whilst the treatment continued, using the method of Williamson-Kligman (Williamson P and Kligman AM, "A New Method for the Quantitative Investigation of Cutaneous Bacteria", J Invest Dermatol 1965 Dec; 45(6): 498-503). The samples were assessed for numbers of coagulase-negative staphylococci (aerobes) on Columbia Agar + 5% Defibrinated Horse Blood (CBA). Bacterial colony counts at a range of dilutions were used to calculate the numbers of bacteria per cm² of skin.

A decline in the numbers of bacteria present at the skin surface, over the course of the treatment period, would indicate that bacteria were being successfully inactivated not only at the skin surface but also in the follicles.

The results are shown in Tables 6a to 6c below, as sampled on the forehead, left cheek and right cheek respectively. "TNTC" means too numerous to count.

**Table 6a (forehead)**

| **Sample point (day)** | **Culony count (per dilution)** | | | | | **Bacteria cm⁻² skin** | **Log10 bacteria cm⁻² skin** |
|---|---|---|---|---|---|---|---|
| | **N** | **10⁻¹** | **10⁻²** | **10⁻³** | **10⁻⁴** | | |
| 0 | TNTC | TNTC | 312 | 28 | 4 | 1.27 × 10⁵ | **5.10** |
| 2 | TNTC | 75 | 3 | 0 | 0 | 3.05 × 10³ | **3.49** |
| 7 | TNTC | 184 | 7 | 1 | 0 | 7.49 × 10² | **2.87** |
| 10 | 247 | 20 | 1 | 0 | 0 | 1.01 × 10³ | **3.00** |
| 14 | 100 | 10 | 1 | 0 | 0 | 4.07 × 10² | **2.61** |
| 14 (+4h) | 37 | 3 | 0 | 0 | 0 | 1.51 × 10² | **2.18** |

**Table 6b left check)**

| **Sample point (day)** | **Colony count (per dilution)** | | | | | **Bacteria cm⁻² skin** | **Log¹⁰ bacteria cm⁻² skin** |
|---|---|---|---|---|---|---|---|
| | **N** | **10⁻¹** | **10⁻²** | **10⁻³** | **10⁻⁴** | | |
| 0 | TNTC | TNTC | 144 | 10 | **3** | 5.87 × 10⁴ | **4.77** |
| 2 | TNTC | 37 | 3 | 0 | 0 | 1.51 × 10³ | **3.18** |
| 7 | 172 | 128 | 2 | 0 | 0 | 7.01 × 10² | **2.85** |
| 10 | 132 | 15 | 1 | 0 | 0 | 5.38 × 10² | **2.73** |
| 14 | TNTC | 40 | 3 | 0 | 0 | 1.63 × 1.0³ | **3.21** |
| 14 (+4h) | 63 | 4 | 0 | 0 | 0 | 2.57 × 10² | **2.41** |

**Table 6c (right cheek)**

| **Sample point (day)** | **Colony count (per dilution)** | | | | | **Bacteria cm⁻² skin** | **Log10 bacteria cm⁻² skin** |
|---|---|---|---|---|---|---|---|
| | **N** | **10⁻¹** | **10⁻²** | **10⁻³** | **10⁻⁴** | | |
| 0 | TNTC | TNTC | 148 | 20 | 2 | 6.03 × 10⁴ | **4.78** |
| 2 | TNTC | 44 | 4 | 0 | 0 | 1.79 × 10³ | **3.25** |
| 7 | 95 | 10 | 7 | 0 | 0 | 3.78 × 10² | **2.59** |
| 10 | TNTC | 40 | 5 | 0 | 0 | 1.63 × 10² | **3.21** |
| 14 | 138 | 12 | 6 | 0 | 0 | 5.62 × 10² | **2.75** |
| 14 (+4h) | 30 | 3 | 0 | 0 | 0 | 1.22 × 10² | **2.09** |

It can be seen from the Table 6 data that the formulation of the invention is effective *in vivo* as an antimicrobial agent. It appears to be reaching the targeted follicles and once there, to be successfully acting as an antimicrobial agent, significantly reducing the numbers of bacteria present at the skin surface following treatment. The formulation is therefore suitable for use as a topical antimicrobial agent.

### Example 7 - in vitro activity against Propionibacterium spp

The following experiments used *P. acnes* NCTC 737 as the test organism.

DDA assays, as described above, were carried out using as the test formulation the gel prepared in Example 2B above. Also tested was the Example 2B gel minus the Klucel™ thickener, which was also subjected to MIC and MBC assays. For the DDAs, which were performed in triplicate, 100 µl of the relevant formulation was loaded onto each disc, and zones of inhibition were recorded after 3 days' anaerobic incubation at 37°C. The MIC and MBC tests were performed in duplicate and the results recorded after 3 days and 7 days respectively, post-subculture onto drug-free medium.

The results are shown in Table 7 below.

**Table 7**

| ***Test formulation*** | ***DDA zone diameter (mm)*** | ***MIC (µg*/*ml)*** | ***MBC (µg*/*ml)*** |
|---|---|---|---|
| Example 2 | >90 mm* | Not tested | Not tested |
| Example 2 minus Klucel™ | >90 mm* | 0.12** | 1.95** |

| | | | |
|---|---|---|---|
| *A value of >90 mm indicates no growth on the entire plate. ** Based on 1% copper usnate in the overall formulation. | | | |

It can be seen from Table 7 that a formulation according to the invention is active as an antibacterial agent against *P. acnes* NCTC 737. This indicates the likely activity of the formulation as an anti-acne agent, the propionibacteria being implicated in acne.

### Example 8 - in vitro activity against P. acnes

The compounds copper (II) usnate (Just for Today Ltd), sodium usnate (Yick-Vic Chemicals) and usnic acid (Sigma-Aldrich) were tested against *P. acnes* NCTC 737, using MIC, MBC and DDA assays as described above. For copper usnate and usnic acid, supplemented DDA assays were also carried out. DMSO was used as the solvent for all three test compounds. For the DDAs, 200 µg of the relevant test compound was loaded onto each disc. The MIC and MBC assays were conducted in triplicate, as were the DDAs for copper usnate.

The results are shown in Table 8 below.

**Table 8**

| ***Test compound*** | ***MIC (µm*/*ml)*** | ***MBC (µg*/*ml)*** | ***DDA Zone diameter (mm ± SD)*** | | |
|---|---|---|---|---|---|
| | | | ***Unsupplemented medium*** | **+*1% NaCl*** | **+*1*% *triolein*** |
| Copper usnate | 0.49 | 1.95 | 46.7 ± 0.4 | 42.9 ± 0.4 | 27.4 ± 1.5 |
| Sodium usnate | 0.49 | 0.98 | 57.9 | Not tested | Not tested |
| Usnic acid | 0.49 | 31.25 | 23.9 | 29.8 | 10.9 |

The Table 8 data indicate that a formulation according to the invention, containing usnic acid or an usnate salt, is likely to be active as an antibacterial agent against *P*. *acnes* NCTC 737. This indicates the likely activity of the formulation as an anti-acne agent, the propionibacteria being implicated in acne. It is of note that the activities of the test compounds appear to be at least partially retained in the presence of lipid and in particular salt.

This example illustrates the suitability of usnic acid and usnates for inclusion in an anti-acne formulation according to the invention. The components (a) to (c) of such a formulation, and if present also component (d), can help to optimise delivery of these highly effective antimicrobial agents to the desired sites on topical application to the skin.

### Example 9 - activity against other Propionibacterium spp

The activity (MIC by agar dilution) of copper usnate was determined against a panel of different propionibacterium strains. The culture medium was Wilkins-Chalgren agar, at pH 6.0. DMSO was used as the solvent. All tests were performed in triplicate. The results are shown in Table 9 below; the resistance phenotype for each of the test species/strains is also indicated.

**Table 9**

| ***Propionibacterial species*** | ***Reference number*** | ***Mutation*/ *gene*** | ***Resistance phenotype*** | ***MIC (µg*/*ml)*** |
|---|---|---|---|---|
| *P. acnes* | NCTC 737 | None | None | 0.49 |
| *P*. *granulosum* | NCTC 11865 | None | None | 1.95 |
| *P. acnes* | PRP-002 | 1058/2058 | Tet/MLS | 0.49 |
| *P. acnes* | PRP-003 | 1058 | Tet | 0.49 |
| *P. acnes* | PRP-004 | 1058 | Tet | 0.49 |
| *P. granulosum* | PRP-005 | Erm X | MLSK | 1.95 |
| *P. granulosum* | PRP-006 | 2058 | MLS | 1.95 |
| *P. acnes* | PRP-007 | Unknown | Clin | 0.49 |
| *P. acnes* | PRP-008 | Unknown | Clin | 1.95 |
| *P. acnes* | PRP-010 | Erm X | MLSK | 0.49 |
| *P. acnes* | PRP-017 | 2058 | MLS | 0.49 |
| *P. granulosum* | PRP-019 | ErmX | MLSK | 1.95 |
| *P. granulosum* | PRP-021 | 2058 | MLS | 1.95 |
| *P. acnes* | PRP-023 | Erm X | MLSK | 0.49 |
| *P. acnes* | PRP-026 | 2059 | MLS | 0.49 |
| *P. acnes* | PRP-039 | 2059 | Tet/MLS | 0.49 |
| *P. granulosum* | PRP-043 | 2059 | MLS | 1.95 |
| *P. granulosum* | PRP-044 | 2059 | MLS | 1.95 |
| *P. acnes* | PRP-046 | None | None | 0.49 |
| *P. acnes* | PRP-053 | 1058/2058 | Tet/MLS | 1.95 |
| *P. granulosum* | PRP-055 | None | None | 1.95 |
| *P. acnes* | PRP-059 | 2058 | MLS | 0.49 |
| *P. acnes* | PRP-068 | 2057 | Ery | 1.95 |
| *P. acnes* | PRP-101 | 1058/2059 | Tet/MLS | 0.49 |
| *P. acnes* | PRP-102 | 1058/2059 | Tet/MLS | 0.49 |

It can be seen from Table 9 that cutaneous propionibacteria, including both antibiotic susceptible and resistant strains, are uniformly susceptible to the usnate salt. This further indicates the utility of formulations containing an usnate either to treat or to prevent infections associated with such bacteria, in particular acne. The results are likely to be of particular clinical value for the antibiotic resistant test strains.

### Example 10 - in vitro activity against staphylococci

Example 8 was repeated (except for the supplemented DDA assays), using *S*. *aureus* ATCC 29213 as the test organism. Copper usnate was also tested against *S*. *epidermidis* NCTC 11047. In the DDA assays, 200 µg of each compound was loaded onto each disc: the culture medium was Mueller-Hinton agar at pH 7.0, and results were recorded after overnight incubation at 37°C in air. The MICs and MBCs were performed in Mueller-Hinton broth at pH 7.0. All tests were carried out in triplicate other than the DDA for sodium usnate, which was carried out on a single plate.

The results are shown in Tables 10 and 11 below, for the *S*. *aureus and S. epidermidis* respectively.

**Table 10 (S. aureus ATCC 29213)**

| ***Compound*** | ***MIC (µg*/*ml)*** | ***MBC (µg*/*ml)*** | ***DDA zone diameter (mm ± SD)*** |
|---|---|---|---|
| Copper usnate | 7.8 | 62.5 | 14.8 ± 0.3 |
| Sodium usnate | 7.8 | 250 | 15.0 |
| Usnic acid | 31.25 | 250 | 13.6 ± 0.3 |

**Table 11 (S. epidermidis NCTC 11047)**

| ***Compound*** | ***MIC (µg*/*ml)*** | ***MBC (µg*/*ml)*** | ***DDA zone diameter (mm ± SD)*** |
|---|---|---|---|
| Copper usnate | 0.98 | 62.5 | 25.7 ± 0.6 |

Tables 10 and 11 show that a formulation according to the invention, containing usnic acid or an usnate salt, is likely to be active as an antibacterial agent against staphylococci. This indicates its likely utility in the treatment (including prevention) of staphylococcal infections such as MRSA.

This example illustrates the suitability of usnic acid and usnates for inclusion in an antimicrobial formulation according to the invention. Again the components (a) to (c) of such a formulation, and if present also component (d), can help to optimise delivery of these highly effective antimicrobial agents to the desired sites on topical application to the skin.

### Example 11 - in vitro activity against corynebacteria

Example 8 was repeated (except for the supplemented DDA assays), using *C. Striatum* NCTC 764 as the test organism. The MICs and MBCs were conducted in triplicate, and the DDAs on single plates.

The results are shown in Table 12 below.

**Table 12 (C. striatum NCTC 764)**

| ***Compound*** | ***MIC (µg*/*ml)*** | ***MBC (µg*/*ml)*** | ***DDA zone diameter (mm)*** |
|---|---|---|---|
| Copper usnate | 3.9 | 62.5 | Not tested |
| Sodium usnate | 3.9 | 125 | 20.0 |
| Usnic acid | 7.8 | 62.5 | 20.0 |

These data show that a formulation according to the invention, containing usnic acid or an usnate salt, is likely to be active as an antibacterial agent against *C*. *striatum* NCTC 764. This indicates its likely utility in the treatment (including prevention) of body odour, in which such bacteria are implicated.

### Example 12 - in vitro activity against S. mutans

Example 11 was repeated, for copper usnate and usnic acid only, using *S. mutans* ATCC 25175 as the test organism. For the DDAs, 200 µg of the relevant compound was loaded onto each disc; the culture medium was Wilkins-Chalgren Anaerobe medium + 1g/L glucose (agar and broth), and results were recorded after 48 hours' incubation at 37°C in air, at pH 7 (± 0.2), with 5% carbon dioxide. The MICs and MBCs were performed in Wilkins-Chalgren broth at pH 7 (± 0.2). The MICs and MBCs were conducted in triplicate, and the DDAs on single plates.

The results are shown in Table 13 below.

**Table 13 (S. mutans ATCC 25175)**

| ***Compound*** | ***MIC (µg*/*ml)*** | ***MBC (µg*/*ml)*** | ***DDA zone diameter (mm)*** |
|---|---|---|---|
| Copper usnate | 1.95 | 31.25 | 32.4 |
| Usnic acid | 3.9 | 125 | 31.2 |

These data show that a formulation according to the invention, containing usnic acid or an usnate salt, is likely to be active as an antibacterial agent against S. *mutans* ATCC 25175. This indicates its likely utility as an agent against oral health problems with which such bacteria are associated, in particular dental caries.

### Example 13 - in vitro activity against S. pyogenes

Example 9 was repeated, for copper usnate only, using *S. pyogenes* ATCC 12344 as the test organism. For the DDAs, 200 µg of the compound was loaded onto each disc; the culture medium was Mueller-Hinton agar at pH 7.0, and results were recorded after 48 hours' incubation at 37°C in air with 5% carbon dioxide. The MICs and MBCs were performed in Mueller-Hinton broth at pH 7.0. All tests were carried out in triplicate.

The results are shown in Table 14 below.

**Table 14 (S. pyogenes ATCC 12344)**

| ***Compound*** | ***MIC (µg*/*ml)*** | ***MBC (µg*/*ml)*** | ***DDA zone diameter (mm ±SD)*** |
|---|---|---|---|
| Copper usnate | 0.98 | 0.98 | 31.3 ± 0.6 |

These and the Example 12 data show that a formulation according to the invention, containing usnic acid or an usnate salt, is likely to be active as an antibacterial agent against streptococci. This indicates its likely utility in the treatment (including prevention) of streptococcal infections, for instance of skin, soft tissue, burns or wounds.

### Example 14 - topical anti-acne formulations

The results from Examples 6 to 9 show that a formulation according to the invention can be effective as a topical antimicrobial agent and as an anti-acne agent. A topical formulation for use in treating acne may thus be prepared by combining usnic acid, or an usnate such as copper usnate, with a suitable primary solvent such as DMI, a glycol, a hydrophobic fatty acid, fatty alcohol or derivative thereof, and optionally an alkylene glycol derivative such as Transcutol™. The formulation may be prepared as described in Examples 1 to 4. It may contain conventional additives, as described above.

The formulation may for example take the form of a cream, lotion, foam, ointment or gel, and it is suitably administered topically to acne-affected skin.

### Example 15 - anti-staphylococcal formulations

The results from Examples 6 and 10 show that a formulation according to the invention can be an effective topical anti-staphylococcal agent. A topical formulation for use against staphylococci such as *S. aureus* may thus be prepared by combining usnic acid, or an usnate such as copper usnate, with a suitable primary solvent such as DMI, a glycol, a hydrophobic fatty acid, fatty alcohol or derivative thereof, and optionally an alkylene glycol derivative such as Transcutol™. The formulation may be prepared as described in Examples 1 to 4, however the ingredients may in this case be formulated as a spray, for instance for application to work surfaces or surgical instruments; as a cleansing gel or lotion for instance for hand washing; as a nasal spray for application to the anterior nares; as ear or eye drops; or in many other appropriate forms.

Such a formulation may in particular be used prophylactically, for instance to reduce the risk of outbreaks ofMRSA or similar infections. It may be applied to the nares, ears or hands, or it may be used to disinfect non-living surfaces.

Similar formulations can be prepared for use in the treatment (including prevention) of skin and soft tissue infections or of infections of wounds and burns, in particular infections caused by staphylococci and/or streptococci and/or other bacteria such as E. *faecalis.* Such formulations may for example be applied via a suitable substrate such as a skin dressing.

### Example 16 - topical anti-BO formulations

Examples 6 to 9 and 11 show that a formulation according to the invention can be active against both propionibacteria and bacteria of the genus *Corynebacterium,* both of which can be associated with body odour. This activity can be of use in preparing antimicrobial formulations, suitably for topical application to the skin, for use against body odour, in particular in the axilla and/or feet.

A topical formulation for use in the treatment of body odour may be prepared by combining usnic acid, or an usnate such as copper usnate, with a suitable primary solvent such as DMI, a glycol, a hydrophobic fatty acid, fatty alcohol or derivative thereof, and optionally an alkylene glycol derivative such as Transcutol™, for instance as described in Examples 1 to 4. The formulation may be prepared and administered using known techniques. It may for example take the form of a roll-on, spray or "stick" anti-perspirant or deodorant formulation, or of a dusting powder such as a talcum powder, or of a gel or cream or ointment. It may contain an anti-perspirant and/or deodorant agent, and/or a fragrance. It may be coated on or incorporated into a sock or shoe, or a shoe insole.

## Claims

1. An antimicrobial and/or anti-acne formulation containing either usnic acid or an usnate, together with: (a) a primary solvent or solvent system in which the usnic acid or usnate is at least partially dissolved; (b) a glycol; and (c) a hydrophobic component (c) selected from fatty acids, fatty alcohols, fatty acid esters, and mixtures thereof, wherein the formulation is in the form of a liquid and contains 1% w/w or less of water.

2. A formulation according to claim 1, which also contains (d) an alkylene glycol ether.

3. A formulation according to claim 1 or claim 2, wherein the primary solvent or solvent system (a) is selected from dimethyl isosorbide (DMI), acetone, methyl ethyl ketone, diethyl ketone, butanone, 3-pentanone, ethanol, phenoxyethanol, isopropyl alcohol, 1-methoxy-2-propanol, benzyl alcohol, tetrahydrofurfuryl alcohol, polyethylene glycols, hexaethylene glycol, polyethoxylated esters, propylene phenoxytol, dimethyl formamide (DMF), dimethyl sulphoxide (DMSO), and mixtures thereof.

4. A formulation according to any one of the preceding claims, wherein the glycol (b) is selected from propylene and butylene glycols and mixtures thereof.

5. A formulation according to any one of the preceding claims, wherein the component (c) is a fatty acid ester.

6. A formulation according to any one of the preceding claims, which additionally contains one or more thickeners or gelling agents.

7. A formulation according to any one of the preceding claims, wherein the usnic acid or usnate active is copper usnate.

8. A formulation according to any one of the preceding claims, which is suitable for topical application to the skin.

9. A product which incorporates an antimicrobial and/or anti-acne formulation according to any one of the preceding claims.

10. An antimicrobial and/or anti-acne formulation according to any one of claims 1 to 8, for use in the treatment of a condition affecting the human or animal body, which condition is caused by, transmitted by and/or exacerbated by microbial activity.

11. An antimicrobial and/or anti-acne formulation according to any one of claims 1 to 8, for use according to claim 10, wherein the condition is selected from acne, a staphylococcal infection and body odour.

## Patentansprüche

1. Antimikrobielle und/oder Anti-Akne-Formulierung, welche entweder Usninsäure oder ein Usnat zusammen mit: (a) einem primären Lösungsmittel oder Lösungsmittelsystem, in welchem die Usninsäure oder das Usnat zumindest teilweise gelöst ist; (b) einem Glykol; und (c) einen hydrophoben Bestandteil (c), ausgewählt aus Fettsäuren, Fettalkoholen, Fettsäureestern und Mischungen davon, enthält, worin die Formulierung in Form einer Flüssigkeit vorliegt und einen Massenanteil von 1 % oder weniger Wasser enthält.

2. Formulierung nach Anspruch 1, welche ferner (d) einen Alkylenglykolether enthält.

3. Formulierung nach Anspruch 1 oder 2, worin das primäre Lösungsmittel oder Lösungsmittelsystem (a) ausgewählt ist aus Dimethylisosorbid (DMI), Aceton, Methylethylketon, Diethylketon, Butanon, 3-Pentanon, Ethanol, Phenoxyethanol, Isopropylalkohol, 1-Methoxy-2-propanol, Benzylalkohol, Tetrahydrofurfurylalkohol, Polyethylenglykolen, Hexaethylenglykol, polyethoxylierten Estern, Propylenphenoxytol, Dimethylformamid (DMF), Dimethylsulfoxid (DMSO) und Mischungen davon.

4. Formulierung nach einem der vorhergehenden Ansprüche, worin das Glykol (b) ausgewählt ist aus Propylen- und Butylenglykolen und Mischungen davon.

5. Formulierung nach einem der vorhergehenden Ansprüche, worin der Bestandteil (c) ein Fettsäureester ist.

6. Formulierung nach einem der vorhergehenden Ansprüche, welche zusätzlich ein oder mehrere Verdickungs- oder Geliermittel enthält.

7. Formulierung nach einem der vorhergehenden Ansprüche, worin die aktive Usninsäure oder das aktive Usnat Kupferusnat ist.

8. Formulierung nach einem der vorhergehenden Ansprüche, welche für die topische Anwendung auf der Haut geeignet ist.

9. Produkt, welches eine antimikrobielle und/oder Anti-Akne-Formulierung gemäß einem der vorhergehenden Ansprüche enthält.

10. Antimikrobielle und/oder Anti-Akne-Formulierung nach einem der Ansprüche 1 bis 8 zur Verwendung bei der Behandlung einer Erkrankung des menschlichen oder tierischen Körpers, welche durch mikrobielle Aktivität verursacht, übertragen und/oder verstärkt wird.

11. Antimikrobielle und/oder Anti-Akne-Formulierung nach einem der Ansprüche 1 bis 8 zur Verwendung nach Anspruch 10, wobei die Erkrankung ausgewählt ist aus Akne, Staphylokokken-Infektion und Körpergeruch.

## Revendications

1. Formulation antimicrobienne et/ou anti-acné contenant soit de l'acide usnique, soit de l'usnate, conjointement avec : (a) un solvant primaire ou un système de solvant dans lequel l'acide usnique ou l'usnate est au moins partiellement dissous ; (b) un glycol ; et (c) un composant hydrophobe (c) choisi parmi les acides gras, les alcools gras, les esters d'acide gras et leurs mélanges, la formulation étant sous la forme d'un liquide et contenant 1 % p/p, ou moins, d'eau.

2. Formulation selon la revendication 1, qui contient également (d) un éther d'alkylène glycol.

3. Formulation selon la revendication 1 ou la revendication 2, dans laquelle le solvant primaire ou le système de solvant (a) est choisi parmi l'isosorbide de diméthyle (DMI), l'acétone, la méthyléthylcétone, la diéthyl-cétone, la butanone, la 3-pentanone, l'éthanol, le phénoxyéthanol, l'alcool isopropylique, le 1-méthoxy-2-propanol, l'alcool benzylique, l'alcool tétrahydrofurfurylique, les polyéthylène glycols, l'hexaéthylène glycol, les esters polyéthoxylés, le propylène phénoxytol, le diméthylformamide (DMF), le diméthylsulfoxyde (DMSO) et leurs mélanges.

4. Formulation selon l'une quelconque des revendications précédentes, dans laquelle le glycol (b) est choisi parmi le propylène et les butylène-glycols et leurs mélanges.

5. Formulation selon l'une quelconque des revendications précédentes, dans laquelle le composant (c) est un ester d'acide gras.

6. Formulation selon l'une quelconque des revendications précédentes, qui contient en outre un ou plusieurs épaississants ou agents gélifiants.

7. Formulation selon l'une quelconque des revendications précédentes, dans laquelle l'actif acide usnique ou usnate est l'usnate de cuivre.

8. Formulation selon l'une quelconque des revendications précédentes, qui est appropriée pour l'application topique sur la peau.

9. Produit qui comprend une formulation antimicrobienne et/ou anti-acné selon l'une quelconque des revendications précédentes.

10. Formulation antimicrobienne et/ou anti-acné selon l'une quelconque des revendications 1 à 8, pour son utilisation dans le traitement d'une affection du corps humain ou animal, ladite affection étant provoquée par, transmise par et/ou exacerbée par une activité microbienne.

11. Formulation antimicrobienne et/ou anti-acné selon l'une quelconque des revendications 1 à 8, pour son utilisation selon la revendication 10, dans laquelle l'affection est choisie parmi l'acné, une infection à staphylocoque et une odeur corporelle.
